# EUROPEAN PATENT APPLICATION

(11) **EP 3 741 369 A1**
(43) Date of publication of application: **25.11.2020**
(21) Application number: 19741419.6
(22) Date of filing: 22.01.2019
(51) Int. Cl.: A61K 31/407, A61K 31/135, A61P 29/00, A61P 25/04

(54) **SYNERGISTIC PHARMACEUTICAL COMBINATION OF THE ACTIVE ENANTIOMER S-KETOROLAC TROMETHAMINE AND TRAMADOL CHLORHYDRATE**

(30) Priority: 22.01.2018 MX 2018000963
(71) Applicant: Amezcua Amezcua, Federico, 28006 Madrid (ES); Amezcua Amezcua, Carlos, 28006 Madrid (ES)
(72) Inventor: GARCÍA ARMENTA, Patricia del Carmen, Esencia Zapopan, Jalisco, 45019 (MX)
(74) Representative: Gonzalez Palmero, Fé
(86) International application number: PCT/MX2019/000003
(87) International publication number: WO 2019/143234

(57) **Abstract**

The present invention relates to a pharmaceutical composition comprising the synergistic combination of a non-steroidal anti-inflammatory drug (NSAID), such as the active ingredient s-ketorolac tromethamine, and an opioid agent, such as the active ingredient tramadol chlorhydrate, which ingredients are formulated with pharmaceutically acceptable excipients. The composition is used for the control and treatment of mild and/or moderate and/or severe pain.

## Description

### FIELD OF THE INVENTION

The present invention is related to the pharmaceutical industry technical field, since it is its object to provide a pharmaceutical composition comprising the synergistic combination of a non-steroidal anti-inflammatory drug (NSAID) that is composed of the active enantiomer S-ketorolac tromethamine and an opioid agent that is composed of the active ingredient tramadol chlorhydrate, both of which are formulated with excipients that are pharmaceutically acceptable for administration orally and/or as an injectable. This combination is indicated for the control and treatment of pain.

### BACKGROUND

Pain is a symptom that is present in many pathologies. Its frequency and severity is variable and depends on multiple factors, with prominent examples being the cause or origin of the pain, the type and intensity of the pain, the evolution and perspective of the patient in relation to the pain itself and/or to the causative illness, as well as the magnitude of the response to the therapeutic resources to be employed, that have already been employed, or are available. Other elements such as repercussions on the quality of life of the patient and/or of family members involved in their care, repercussions on the function and affective and emotional aspects of the patient, and particularly the resulting costs for assistance and the patient's ability to pay must be incorporated into this clinical approach. For all of these reasons, regardless of whether acute or chronic pain is involved, pain is an important health problem and is regrettably on the rise, given that, as the average life expectancy of the population increases, the pathologies related to this age are the chronic-degenerative diseases, which are highly disabling or entail high morbidity and mortality.

There has been an increase in the interest in the study of pain in recent years; the discovery of the descending inhibitory nervous pathways that originate in the brainstem and descend through the medulla, modulating the spinal nociceptive activity, represents one great advancement (Fields and Basbaum, 1978).

These descending pathways, like the spinal and supraspinal pathways, respond to opioids and other analgesics, as well as to physiological and experimental stimuli, including stress (Mayer and Liebeskind, 1974).

It has been speculated that the activation of this descending control system might be responsible for the analgesia induced by placebos and of the analgesic effects of acupuncture in some circumstances.

Pain is defined as "an unpleasant sensory and emotional experience associated with actual or potential tissue damage or described in terms of such damage" (International Association for the study of Pain, Subcommittee on Taxonomy, 1979).

Even though the mechanisms and pathways of pain are better known today, it must be said that, besides the activation of the nociceptive pathways, individual perception of pain and the appreciation of its meaning are complex phenomena that involve psychological and emotional aspects (McGrath, 1990).

The perception of pain depends on complex interactions between the nociceptive impulses in the ascending pathways and the activation of descending inhibition systems. Knowing this gives us a basis for a more comprehensive and multimodal focus in the evaluation and treatment of the patients with pain and helps us confirm that clinical experiences that focus on a one-way approach are not the most appropriate.

Therefore, the individual management of pain must consider the state of the illness, concurrent medical conditions, characteristics of the pain, and psychological and cultural characteristics of the patient. A constant evaluation of the pain and the effectiveness of the treatment is also required.

As long as there is pain, the doctor must provide optimal alleviation, with routine evaluations and with one or more kinds of treatments.

The scale of the World Health Organization (WHO) recommends the progressive handling of the doses and the type of analgesic in order to achieve effective pain management. In a global level, more than 30 million people take non-steroidal anti-inflammatory drugs (NSAID) daily, 40% of whom are older than 60. In the treatment of pain, the alternative to a specific analgesic drug is, in general, based on the type of pain.

Even though there are many medications for the treatment of pain, one of the groups employed for the treatment thereof are the non-steroidal anti-inflammatory drugs, which exhibit analgesic, anti-inflammatory and antipyretic activity. This group is composed of: acetylsalicylic acid, diclofenac, diflunisal, etodolac, fenoprofen, flurbiprofen, indomethacin, ketoprofen, ketorolac, meclofenamate, meloxicam, naproxen, piroxicam, sulindac, celecoxib, salicylate, as well as the pharmaceutically acceptable salts. However, the most frequent adverse effects with the use of these active ingredients are directly related to the gastrointestinal tract and the renal or hematological function; they inhibit platelet aggregation and can incite the formation of gastric ulcers.

Another group used for the treatment of pain are the opioid-type analgesics, which comprise: morphine, codeine, thebaine, ethylmorphine, heroin, dihydrocodeine, nalorphine, oxymorphone, oxycodone, nalbuphine, naloxone, naltrexone, etorphine, cyprenorphine, diprenorphine, buprenorphine, levorphanol, levallorphan, butorphanol, dextrorphan, pentazocine, ketocyclazocine, cyclazocine, meperidine, phenazopyridine, profadol, loperamide, diphenoxylate, tilidine, fentanyl, sufentanil, alfentanil, remifentanil, methadone, dextropropoxyphene, dezocine, tramadol, metamizole, as well as pharmaceutically acceptable salts thereof. While the opioid analgesics continue to be the most effective therapy available for the treatment of moderate to severe pain, the problem regarding the collateral effects has persisted.

Therefore, an effective treatment is needed which provides an analgesic effect at a lower dose than the one commonly used, in less time, and with fewer adverse effects is necessary. Consequently, the present invention comprises the combination of S-ketorolac tromethamine and tramadol chlorhydrate for the treatment of mild and/or moderate and/or severe pain.

Ketorolac is a non-steroidal anti-inflammatory agent with analgesic properties that was introduced into clinical practice in 1990 due to its analgesic potency, which is similar to that of morphine and meperidine. Ketorolac belongs to the group of non-steroidal anti-inflammatory drugs (NSAID) (Ong, Tan, 2004). It is a powerful analgesic with moderate anti-inflammatory and antipyretic action.

It has proven to be very useful in the management of acute pain, chiefly during the postoperative period (Catapano, 1996). The oral administration of 10 to 30 mg of ketorolac is considered to be the conventional dose for pain alleviation.

The most frequent adverse effects with the use of ketorolac are directly related with the pharmacological actions of this type of drug. In particular, they produce effects on the gastrointestinal tract and on the renal or hematological functions (Gillis, Brogden, 1997). They inhibit platelet aggregation and can incite the formation of gastric ulcers (Roberts, Morrow, 2003).

The scientific evidence that is currently available shows that the risk of developing severe complications due to peptic ulcer (particularly upper gastrointestinal bleeding) is consistently higher with the use of ketorolac compared to other non-steroidal anti-inflammatory drugs, and the increased risk can be especially significant when used outside of the currently authorized conditions of use.

Ketorolac tromethamine is a racemic mixture of the enantiomers [-] S and [+] R, and the first is the one that possesses the analgesic activity.

S-ketorolac tromethamine is a non-steroidal anti-inflammatory drug (NSAID) from the heterocyclic acetic acid derivatives family, used as an analgesic, an antipyretic and an anti-inflammatory drug. The enantiomer S (-) is the most active; it has been determined that this enantiomer of the racemic mixture is the one that basically has the analgesic effect, which is almost double that of the racemic form and approximately 60 times more potent, thereby enabling the dose to be reduced by up to 50% and thus reducing the risk of severe side effects that are produced due to the chronic consumption of the medications based on the current ketorolac racemic salts.

S-ketorolac tromethamine is the compound:
2-amino-2-hydroximethyl-1,3 propanediol (1:1) S-(-)-5-benzoyl-2,3-dihydro-1H-pyrrolizine-1-carboxylate; or
S-(-)-benzoyl-2,3-dihydro-1 H-pyrrolizine-1-carboxylic acid salt with 2-amino-2-hydroximethyl-1,3-propanediol (1:1),
   represented by the formula (I):

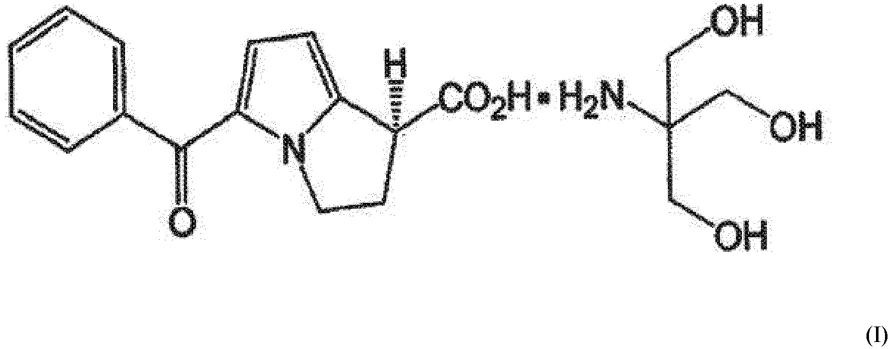

It was described for the first time in US patent 4,089,969 as having activity to treat inflammation, pain, or pyrexia in mammals, and its tromethamine compound was described in the publication "Pharmacology and analgesic, anti-inflammatory profile of ketorolac and its tromethamine salt," (W.H. Rooks et al., Agent Actions, 1982).

Among the opioid-type drugs, tramadol is widely used because, in certain cases, it proves to be an alternative to the use of morphine, the use of which is very limited due to the high incidence of the adverse effects it can present, especially the appearance of a tolerance to the antinociceptive effect, which leads to a gradual increase in the dose administered, up to the point at which these doses pose a considerable risk of the appearance of toxic effects or even death. Tramadol is a compound of synthetic origin that is designed to possess effects comparable to those of morphine to the greatest extent possible without the high incidence of adverse effects associated with the latter.

Tramadol chlorhydrate is a narcotic analgesic that is proposed for severe pain. It is a synthetic analogue of codeine; tramadol chlorhydrate has central analgesic properties with effects similar to those of opioids such as morphine and codeine, acting on the specific opioid receptors. Tramadol chlorhydrate salt is used as a narcotic analgesic for severe pain, can be addictive, and mildly inhibits the reuptake of norepinephrine and serotonin.

Tramadol chlorhydrate is the compound:
(1R,2R)-rel-2-[(dimethylamino)methyl]-1-(3-methoxyphenyl)cyclohexanol
represented by the formula (II):

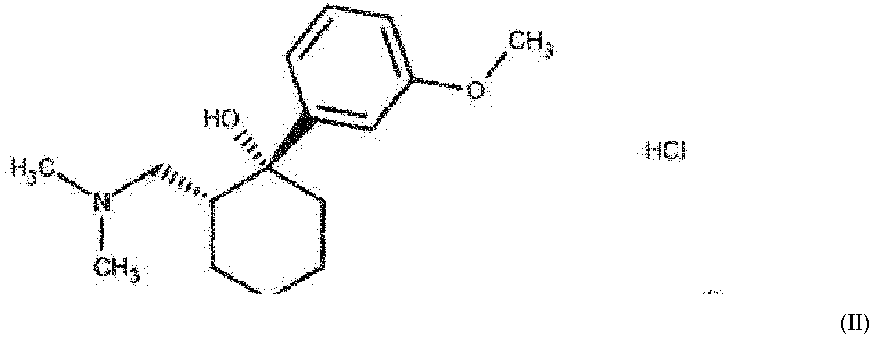

It was described for the first time in US patent 3,652,589 as having high analgesic activity and low toxicity.

The present invention is characterized in that it provides a composition that comprises the combination of an NSAID and an opioid, more specifically the combination of S-ketorolac tromethamine with tramadol chlorhydrate. Currently the combinations of these are commonly used for pain control.

In the prior art, the patent MX266401 describes the pharmaceutical composition for the treatment of pain, characterized in that it comprises: ketorolac tromethamine as a non-steroidal anti-inflammatory drug in an amount of 0.0010 g to 0.1000 g and tramadol chlorhydrate as an opiate analgesic in a quantity of 0.0010 g to 0.20000 g combined with a pharmaceutically acceptable excipient, formulated in a single oral unit-dose; the patent application MX/a/2007/003948 describes the pharmaceutical control that comprises the combination of two active substances, one a non-steroidal anti-inflammatory known as ketorolac, and the other an opioid analgesic known as tramadol; both are formulated to be administered orally as a sublingual tablet, which is indicated for the alleviation and/or treatment of pain of moderate to severe intensity; patent application MX/a/2014/005153 describes the oral pharmaceutical composition of ketorolac tromethamine-tramadol chlorhydrate for allopathic treatment relating to pain (from moderate to severe), analgesic, anti-inflammatory, fever or antipyretic, for oral administration and specifically as a sublingual tablet; patent application MX/a/2014/005152 describes an injectable pharmaceutical composition of the combination of ketorolac tromethamine/tramadol chlorhydrate for allopathic treatment relating to pain (from moderate to severe); it is an analgesic, anti-inflammatory, anti-fever, or antipyretic, *inter alia*, using fewer excipients that do not contribute to the desired therapeutic effect; patent application PA/a/2003/012048 describes the formulation or combination of two compounds, one a non-steroidal anti-inflammatory known as ketorolac and an opioid analgesic known as tramadol, which are formulated in an injectable solution to be administered every 12 hours; patent application MX/a/2010/011193 describes a liquid pharmaceutical composition, in a solution, that is formulated to be administered orally as drops, comprises the synergistic combination of ketorolac and tramadol, as well as pharmaceutically acceptable excipients, and is useful in the prevention and treatment of pain of moderate to severe intensity expressed in pediatric patients without observation of adverse collateral effects; patent application PA/a/2002/010828 describes the formulation or combination of two compounds, one being a non-steroidal anti-inflammatory known as ketorolac and the other an opioid analgesic known as tramadol, which are formulated as capsules; US patent 9,265,732 describes a unitary composition for oral administration that comprises a first drug such as tramadol surrounded by a membrane that delays its release and a second drug such as ketorolac that is released before the first drug, the first drug not being released as a unitary dosage during a period of time equal to or at least one quarter of Tmax2, Tmax2 being the time required for the second medication to reach the maximum plasma concentration when administered to a patient; US patent 6,923,988 describes solid pharmaceutical compositions for improved administration of a wide variety of active pharmaceutical ingredients contained therein or administered separately, selected from ketorolac and tramadol, with the solid pharmaceutical composition including a solid vehicle that contains a substrate and an encapsulation coating over the substrate. The solid composition or the encapsulation coating can include different combinations of active pharmaceutical ingredients, hydrophilic surfactant, and lipophilic and triglyceride surfactants.

The present invention is characterized in that it provides a composition that comprises the combination of S-ketorolac tromethamine and tramadol chlorhydrate, which is not known from the prior art. The potential advantage of using S-ketorolac tromethamine and tramadol chlorhydrate combination therapy is that the analgesic effects can be maximized while the incidence of the adverse effects is minimized.

The use of this combination of medications offers an analgesic synergy that allows for a reduction in the doses required and a reduction in the adverse effects.

### OBJECT OF THE INVENTION

It is the object of the invention to offer a new therapeutic option for the control and treatment of pain that is capable of diminishing the symptomatology and improving patients' quality of life. This is achieved through the strategy of reevaluating a racemic drug such as ketorolac (NSAID) and segregating the beneficial portion, in this case the S enantiomer, to obtain a single isomeric compound that is pure and has a better therapeutic index than the mixture formulated as a racemate. The use of the combination of the active enantiomer S-ketorolac tromethamine with tramadol chlorhydrate generates a synergic interaction, improving its therapeutic potency and onset of action and reducing its adverse effects.

The combination of these active substances as S-(-) ketorolac tromethamine, which is the active enantiomer of the racemic mixture of the ketorolac and the tramadol chlorhydrate, thus provides greater pharmacological potency; based on S-ketorolac tromethamine, it is 3.3 times more potent than rac-ketorolac, 82 times more potent than morphine, and 1037 times more potent than AA, and in combination with tramadol chlorhydrate it improves the therapeutic action, offering the following benefits: administration of lower concentrations of the active substances than when they are administered separately, greater efficacy and greater therapeutic potency, as well as a substantial reduction of the likelihood of collateral effects that may appear when administered independently as compared to when they are administered separately.

### BRIEF DESCRIPTION OF THE FIGURES

FIGURE 1. Temporal course of tramadol chlorhydrate administered orally to a rat.
FIGURE 2. Temporal course of S-ketorolac tromethamine administered orally to a rat.
FIGURE 3. Comparison of dose-response curve of S-ketorolac tromethamine and tramadol chlorhydrate, administered orally to a rat.
FIGURE 4. Temporal course of the combination of S-ketorolac tromethamine and tramadol chlorhydrate, 10 mg/kg.
FIGURE 5. Temporal course of the combination S-ketorolac tromethamine and tramadol chlorhydrate, 17.80 mg/kg.
FIGURE 6. Temporal course of the combination S-ketorolac tromethamine and tramadol chlorhydrate, 31.60 mg/kg.
FIGURE 7. Temporal course of the combination S-ketorolac tromethamine and tramadol chlorhydrate, 56.20 mg/kg.

### DETAILED DESCRIPTION OF THE INVENTION

Nowadays, the strategy for increasing the efficacy of an analgesic treatment and significantly reducing the side effects is pursued through the combined administration of two or more active agents. Combinations for the treatment of pain have proven to be positive. In the present invention, it has been demonstrated with preclinical tests that the innovative combination of S-ketorolac tromethamine and tramadol chlorhydrate in specific doses manifests a surprising and strong synergic therapeutic effect in the treatment of pain. This combination is formulated with pharmaceutically acceptable excipients and indicated for the control and treatment of mild and/or moderate and/or severe pain.

The active agents of the NSAIDs are a group that have the primary effect of inhibiting the synthesis of the prostaglandins, which are responsible for fever, pain, and inflammation, through the inhibition of the cyclooxygenase enzyme. The cyclooxygenase enzyme presents two isoforms called COX-1 and COX-2, coded by different genes with similar chemical structures. The COX-1 isoform is expressed in a constitutive form in most parts of the tissue, while COX-2 is induced by the inflammations. The isoform COX 1 is useful for the maintenance of the normal physiological state of many tissues, including the protection of gastrointestinal mucus, renal blood flow control, homeostasis, autoimmune response, pulmonary functions, as well as of the central nervous system and cardiovascular and reproductive systems. The COX-2 isoform is induced in inflammation by various stimuli: cytokines, endotoxins and growth factors, also creates inductor prostaglandins, which contribute to the development of edema, flush, fever and hyperalgesia.

The consequences of the blocking of COX-1 are the inhibition of the protection of its mucus and an increase in acid secretion, which can lead to erosion, ulceration, perforation, and hemorrhaging at a gastrointestinal level. On the other hand, the selective inhibition of the COX-2 can induce a relative decrease in the endothelial production of prostacyclin; however, the platelet production of thromboxane A2 is not altered, provoking an imbalance and generating an increase in the risk of thrombosis and vascular events.

At a clinical level, NSAID compounds are employed therapeutically to alleviate pain, and many of these are administered in the form of racemic mixture. The agent selected for the present invention is S-ketorolac, which represents an alternative due to its high efficacy and good tolerance at a gastric level. The S-(-)ketorolac is the active enantiomer of the ketorolac racemic mixture, and its development is intended to replace the racemic mixtures by the enantiomerically pure active forms.

Moreover, the opioid-type active agents are characterized by their selective affinity for opioid receptors, achieving high-intensity analgesia that is produced primarily via the CNS. The existence of three specific recognition sites called opioid receptors have been demonstrated: the m, activated by morphine (analgesia, miosis, respiratory depression, bradycardia, hypothermia and indifference toward environmental stimuli), the k, activated by the ketocyclazocine (miosis, general sedation, depression of flexor reflexes, dysphoria, and hallucinosis), and the s, activated by SKF10047 or N-alilnormetazocine (mydriasis, respiratory activation, tachycardia and delirium).

The consequences of the analgesia in the opioid receptors expressed in side effects occur at different levels of the human body and manifest themselves in the form of nausea, vomiting, and constipation on the gastrointestinal side, xerostomia, urinary retention, and postural hypotension in the autonomous nervous system, and are expressed in the central nervous system as sedation, myoclonus cognitive deterioration, hallucinations, delirium, hyperalgesia, convulsions, and at the level of the skin in the form of itchiness and hyperhidrosis.

At a clinical level, tramadol, more specifically tramadol chlorhydrate, is a drug that is used very extensively because it represents an alternative option in certain cases to the use of morphine, the use of which is very limited due to the high incidence of adverse effects that can appear, especially tolerance to the antinociceptive effect, which leads to the gradual increase in the dose administered, up to the point that these doses result in considerable risk of appearance of toxic effects or even death. This active ingredient is a compound of synthetic origin that is designed to possess, to the greatest extent possible, effects that are comparable to those of morphine without the high incidence of adverse effects associated therewith.

The present invention allows for the reduction of side effects that the separate administration of each compound can provoke, using doses that are lower than those employed commercially. The behavior of S-ketorolac tromethamine and tramadol chlorhydrate in combination has been demonstrated preclinically, with the interaction and synergism therebetween having been successfully demonstrated with the optimal combination proportions, as well as a high degree of efficacy and therapeutic potentiation.

### Materials and methods:

### Test animals:

In order to determine the antinociceptive effects, male Wistar rats weighing between 180 and 200 g were employed. These were handled in accordance with the Official Mexican Norm (NOM-062-ZOO-1999) and in accordance with the general principles of lab animal care (NIH publication #85-23, revised in 1985). The minimum number of test animals were used (6 animals per experimental point for antinociceptive effects, motor coordination, and gastrointestinal damage, and 10 animals per experimental point for adverse effects: lethal dose 50). The animals were kept in a room with alternate cycles of light/darkness. Twelve hours before the antinociceptive tests, the food was withdrawn, and they only had free access to water. All of these experiments were conducted during the light phase.

### Active agents used for the experimental design:

Uric acid, S-ketorolac tromethamine, tramadol chlorhydrate, carboxymethyl cellulose, and mineral oil were used. The active agent indomethacin was used as gastrointestinal damage positive control.
The analgesics were dissolved or suspended in carboxymethyl cellulose at 0.5% and administered orally in a volume of 4 ml/kg.

### Pain-induced dysfunction in rat:

This methodology is commonly known as "pain-induced functional impairment model in the rat" (PIFIR). This methodology was used to determine and compare the antinociceptive effects of these analgesics.

The methodology of the PIFIR experimental procedure is briefly described below: The animals were anesthetized in an anesthesia chamber saturated with isoflurane vapors. The gouty arthritis was induced by applying an intra-articular injection (i.art.) with 0.05 ml of uric acid suspended in mineral oil (at 30%) in the right hind limb, exactly in the femur-tibia-patella joint.

A 1 ml glass syringe with a No. 22, 4 mm long needle was used for the i.art. injection. Immediately afterward, an electrode was fixed on each hind limb in the middle of the plantar calluses. After the rats recovered from the anesthesia, they were placed in a 30 cm diameter stainless steel rotating cylinder. The cylinder was rotated at 4 rpm, forcing the rats to walk for 2 minutes every half hour for a total of 6.5 hours (2.5 h to generate the gouty arthritis and 4 h to evaluate the antinociceptive effects of the analgesics). The variable measured was the time of contact of each one of the hind limbs of the rats in the cylinder.

When the electrode and the cylinder make contact, a circuit closes, and the relationship between the time of contact of the extremity to which the uric acid was administered as compared to the one to which uric acid was not administered was registered in a computer. The percentage functionality indexes (FI%) are calculated with these variables. Once the total dysfunction (FI=0%) was produced due to the effect of the uric acid, the corresponding doses of each analgesic were administered individually or in combination.

The uric acid leads to complete dysfunction in the right hind limb by approximately 2.5 hours after administration; this corresponded to a FI% value of zero. At that moment, the rats that received only the vehicle (carboxymethyl cellulose) orally (negative control) did not exhibit any recovery of the FI% during the ensuing 4-hour period. The medium and standard error of the evaluation of the antinociceptive effects are presented in all of the graphics as from time "0," which is the moment at which the arthritis is completely developed in the rat and is the precise moment of the administration of tramadol chlorhydrate and S-ketorolac tromethamine.

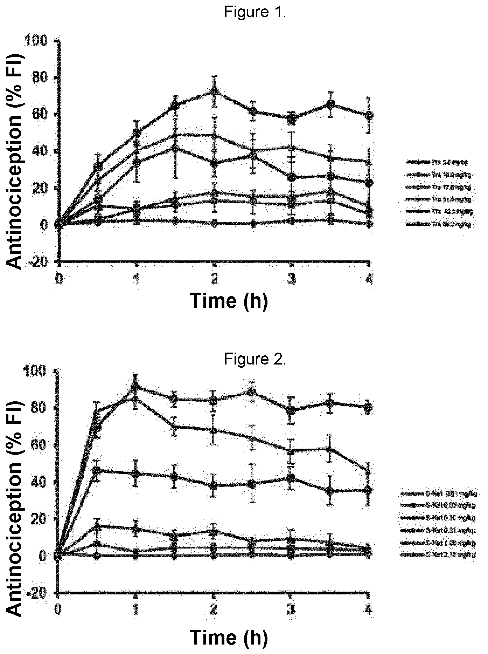

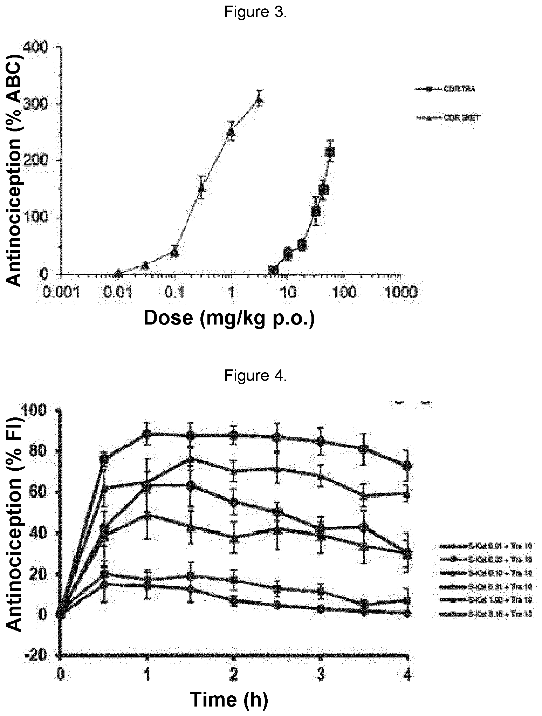

With the administration of these doses, the complete doses-responses of S- ketorolac tromethamine and tramadol chlorhydrate were plotted separately in order to perform a comparative analysis for efficacy and potency. In the case of tramadol chlorhydrate, the curves were obtained through a single and acute 4-hour administration in doses with algorithmic increases (of 5.6 to 56.2 mg/kg), (Figure 1). The S-ketorolac tromethamine temporal curves were obtained through acute administration evaluated for 4 hours in doses with algorithmic increases (from 0.01 to 3.16 mg/kg). (Figure 2). In both cases, in these experimental conditions, doses-dependent antinociceptive effects were observed for 4 hours. From the above, the orally administered antinociceptive pharmacological activity was demonstrated by S- ketorolac tromethamine and tramadol chlorhydrate doses-response curves (Figure 3).

### Antinociceptive efficacy of both active agents:

Table 1 presents the corresponding active analgesic ingredient presented, the dose that generates the efficacy of the drug, the value of the maximum efficacy that this drug is capable of producing under these experimental conditions and which is expressed as the ABC obtained from the respective temporal curve, expressed as the mean with a measurement of dispersion as the standard error. It was thus observed that S-ketorolac tromethamine generates greater efficacy (which is why the relative efficacy is 1.0), while tramadol chlorhydrate exhibited less efficacy - that is, a smaller overall effect (216.66 au) with its higher dose - so the calculated relative efficacy of tramadol chlorhydrate as compared to S-ketorolac tromethamine was 0.70, even though the maximum tramadol chlorhydrate dose administered is quite high (56.2 mg-/kg) compared with those required for S-ketorolac tromethamine.

| Table 1. Pharmacological efficacy | | | |
|---|---|---|---|
| Active agent | Dose (mg/kg,po) | Maximum efficacy (ABC: X ± E.E.) | Relative efficacy |
| S-ketorolac tromethamine | 3.16 | 310.5 ± 13.5 | 1.0 |
| Tramadol chlorhydrate | 56.23 | 216.7 ± 19.1 | 0.70 |

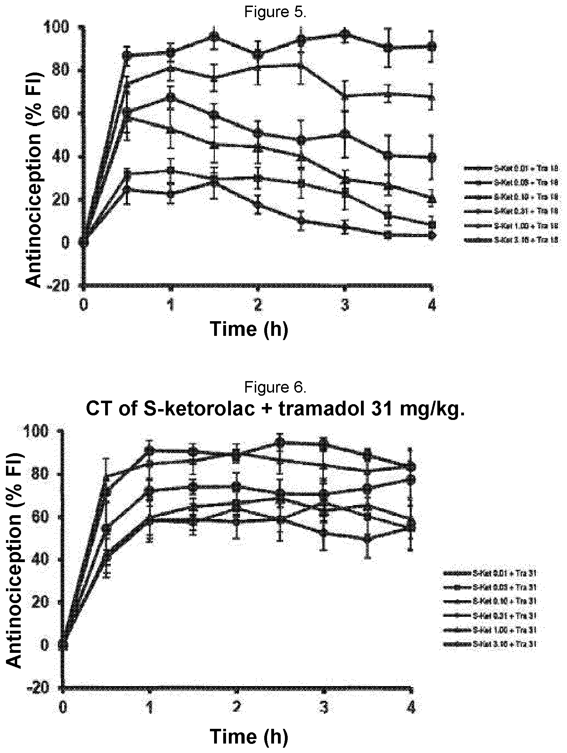

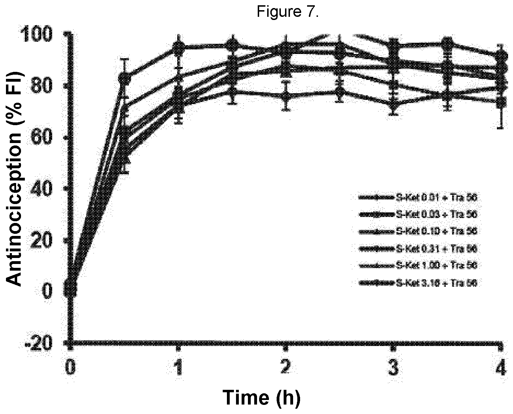

After this, 24 different combinations of S-ketorolac tromethamine and tramadol chlorhydrate were analyzed in order to detect the type of interaction and the most significant combinations due to the degree of efficacy or the degree of antinociceptive potency. It was decided to include the following doses of the S-ketorolac tromethamine doses-response curves for the combinations: 0.01, 0.03, 0.10, 0.31, 1.00, and 3.16 mg/kg; and four different doses of tramadol chlorhydrate: 10.0, 17.8, 31.6, and 56.2 mg/kg; these doses were selected from the tramadol chlorhydrate doses-response curve and comprise the doses located in the linear part of the sigmoid, from a small dose to a sub-maximum dose, (Figures 4, 5, 6, and 7) in order to enable the doses-dependent effects for both active agents to be demonstrated.

Pharmacological treatments for pain exist in the current state of the art; however, there is no treatment that is characterized by the combination of the active agents S-ketorolac tromethamine and tramadol chlorhydrate, so the development of the present invention provides a real and secure alternative for the control and treatment of mild and/or moderate, and/or severe pain, offering an improvement in treatment times, therapeutic effects, and secondary reactions. For each one, an amount of approximately 0.01 mg to approximately 100 mg is administered per treatment day.

The present invention is developed to be administered orally, nasally, intramuscularly, intravenously and topically; with an immediate release for both drugs or with a modified release for one or both drugs, at a lower dose, higher therapeutic potency and reduced risk of adverse events.

### EXAMPLES

Below, as an example, some pharmaceutical compositions are described in a non-limitative manner:
Example 1: Compositions for oral, nasal and/or topic administration:

| |
|---|
| S-ketorolac tromethamine |
| Tramadol chlorhydrate |
| Excipient and/or pharmaceutically accepted vehicle |

Example 2: Composition for intramuscular and intravenous administration:

| |
|---|
| S-ketorolac tromethamine |
| Tramadol chlorhydrate |
| Excipient and/or pharmaceutically accepted vehicle |

The present invention can be represented in other specific ways without straying from its spirit or essential characteristics. The modalities described shall be considered in all aspects, solely as illustrative and not in a restrictive manner. Therefore, the scope of the present invention is indicated by the enclosed claims rather than by the above description. All the changes that are included within the significance and range of equivalence of the claims shall be included within its scope.

Overall, the present invention provides the following advantages:
1. Both S-ketorolac tromethamine and tramadol chlorhydrate generate antinociceptive effects that are dependent on the gouty arthritis-type pain dose in the rat.
2. S-ketorolac tromethamine was more effective than tramadol chlorhydrate, and tramadol had only 70% of the efficacy exhibited by S-ketorolac tromethamine.
3. The interaction between S-ketorolac tromethamine and tramadol chlorhydrate was clearly demonstrated.
4. The analysis of 24 combinations between S-ketorolac tromethamine and tramadol chlorhydrate showed that depending on the proportion of the combination, summation synergism effects and potentiation synergism effects can be generated.
5. The combination of S-ketorolac tromethamine and tramadol chlorhydrate potentiates the desired antinociceptive effects, and the undesired gastrointestinal damage effects are not enhanced, which is very convenient.

## Claims

1. A pharmaceutical synergistic combination, **characterized in that** it comprises:
i. an NSAID agent and/or pharmaceutically accepted salts thereof,
ii. an opioid agent and/or pharmaceutically accepted salts thereof,
iii. a vehicle and/or pharmaceutically accepted excipient,
the combination having been demonstrated in a preclinical trial for oral administration and being indicated for the control and treatment of neuropathic and/or nociceptive pain diseases in mammals.

2. The combination of claim 1, **characterized in that** the NSAID agent is S-ketorolac tromethamine.

3. The combination of claim 1, **characterized in that** the opioid agent is tramadol chlorhydrate.

4. The combination of claim 1, **characterized in that** S-ketorolac tromethamine is present in a concentration of from approximately 0.01 to approximately 100 mg.

5. The combination of claim 1, **characterized in that** the active agent tramadol chlorhydrate is present in a concentration of from approximately 0.01 to approximately 100 mg.

6. The combination of claim 1, useful for the preparation of a medication for analgesic and/or anti-inflammatory and/or antipyretic therapy.

7. The combination as set forth in claim 1 and 6, wherein the medication is useful for the control and/or treatment of moderate pain in mammals.

8. The combination of claim 1, wherein the mammal is a human being or an animal.

9. A treatment method for neuropathic and/or nociceptive pain which comprises the administration to mammals suffering from this disease of an effective amount of the combination of at least one compound that is selected from the group of the NSAIDs and at least one compound that is selected from the group of the opioids.

10. The treatment method for neuropathic and/or nociceptive pain as set forth in claim 9, wherein the NSAID compound is selected from S-ketorolac tromethamine.

11. The treatment method for neuropathic and/or nociceptive pain as set forth in claim 10, wherein the opioid analgesic compound is selected from tramadol chlorhydrate.

12. The treatment method for neuropathic and/or nociceptive pain as set forth in claims 10 and 11, wherein the compounds are administered orally, nasally, topically, intramuscularly, and/or intravenously.

13. The treatment method for neuropathic and/or nociceptive pain as set forth in claim 12, wherein the compounds of the administration is a quantity of from approximately 0.01 mg to approximately 100 mg per day.
